# EUROPEAN PATENT APPLICATION

(11) **EP 4 505 958 A1**
(43) Date of publication of application: **12.02.2025**
(21) Application number: 24192260.8
(22) Date of filing: 01.08.2024
(51) Int. Cl.: A61B 18/14, A61B 34/30, A61B 90/00

(54) **SURGICAL ROBOTIC SYSTEM AND METHOD FOR DEPLOYING A PROBE OF A MULTIFUNCTION SURGICAL INSTRUMENT**

(30) Priority: 03.08.2023 US 202363517376 P; 01.07.2024 US 202418760279
(71) Applicant: Covidien LP, Mansfield, MA 02048 (US)
(72) Inventor: LOSCHAK, Paul M., Boston, 02210 (US); COCKERELL, Jullian C., Boston, 02210 (US)
(74) Representative: Maschio & Soames IP Ltd

(57) **Abstract**

A surgical system includes a surgical instrument which includes first and second jaw members where at least one of the first or second jaw members is movable relative to the other of the first or second jaw members from a spaced apart position to an approximated position to grasp tissue therebetween. At least one of the first or second jaw members is also adapted to connect to a source of energy for conducting energy through tissue grasped between the first and second jaw members to treat tissue. The surgical instrument also includes a probe adapted to connect to a source of energy for conducting energy through tissue in contact with the probe to treat tissue. The probe is movable from a retracted position to a deployed position. The system also includes a camera configured to capture a video of a tissue and the surgical instrument and a monitor configured to display the video of the tissue and the surgical instrument, and a virtual representation of the probe in the deployed position.

## Description

### CROSS-REFERENCE TO RELATED APPLICATIONS

This application claims the benefit of, and priority to, U.S. Provisional Patent Application Serial No. 63/517,376 filed on August 3, 2023. The entire contents of the foregoing application is incorporated by reference herein.

### FIELD

This disclosure relates to surgical instruments and systems and, more particularly, to multifunction surgical instruments such as for use in surgical robotic systems.

### BACKGROUND

Robotic surgical systems are increasingly utilized in various surgical procedures. Some robotic surgical systems include a console supporting a robotic arm. One or more different surgical instruments may be configured for use with the robotic surgical system and selectively mountable to the robotic arm. The robotic arm provides one or more inputs to the mounted surgical instrument to enable operation of the mounted surgical instrument, e.g., to rotate, articulate, and/or actuate the mounted surgical instrument.

As can be appreciated, as additional functional components are added to surgical instruments for use in surgical robotic systems, additional actuation structures, deployable components, and/or electrical connections are required. These additional structures, components, and/or connections may present challenges with respect to spatial constraints and/or mechanical features of the surgical instruments.

### SUMMARY

As used herein, the term "distal" refers to the portion that is being described which is farther from an operator (whether a human surgeon or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, design variations, and/or other variations, up to and including plus or minus 10 percent. Further, to the extent consistent, any of the aspects described herein may be used in conjunction with any or all of the other aspects described herein.

According to one embodiment of the present disclosure, a surgical system is disclosed. The surgical system includes a surgical instrument which includes first and second jaw members where at least one of the first or second jaw members is movable relative to the other of the first or second jaw members from a spaced apart position to an approximated position to grasp tissue therebetween. At least one of the first or second jaw members is also adapted to connect to a source of energy for conducting energy through tissue grasped between the first and second jaw members to treat the tissue. The surgical instrument also includes a probe adapted to connect to a source of energy. The probe is movable from a retracted position to a deployed position. The system also includes a camera configured to capture a video of a tissue and the surgical instrument. The system further includes a controller configured to generate a virtual representation of the probe based on the video of the surgical instrument and a monitor configured to display the video of the tissue and the surgical instrument, and a virtual representation of the probe in the deployed position.

Implementations of the above embodiment may include one or more of the following features. According to one aspect of the above embodiment, the surgical system may also include a switch operable in a first stage in response to which the probe is moved to the deployed position. The switch may be also operable in a second stage in response to which the probe is energized. The controller may be further configured to analyze the video to identify a critical structure of the tissue. The controller may be further configured to output the virtual representation of the probe based on a distance of the probe to the critical structure. The controller may be further configured to highlight the critical structure. The monitor may be also configured to display the virtual representation in response to a user command.

According to another embodiment of the present disclosure, a surgical robotic system is disclosed. The surgical robotic system includes a first robotic arm controlling a surgical instrument which includes first and second jaw members where at least one of the first or second jaw members is movable relative to the other of the first or second jaw members from a spaced apart position to an approximated position to grasp tissue therebetween. At least one of the first or second jaw members is also adapted to connect to a source of energy for conducting energy through tissue grasped between the first and second jaw members to treat the tissue. The surgical instrument also includes a probe adapted to connect to a source of energy. The probe is movable from a retracted position to a deployed position. The system also includes a second robotic arm controlling a camera configured to capture a video of a tissue and the surgical instrument. The system further includes a controller configured to generate a virtual representation of the probe based on the video of the surgical instrument and a monitor configured to display the video of the tissue and the surgical instrument, and a virtual representation of the probe in the deployed position.

Implementations of the above embodiment may include one or more of the following features. According to one aspect of the above embodiment, the surgical robotic system may further include a surgical console having a foot pedal operable in a first stage in response to which the probe is moved to the deployed position. The foot pedal is operable in a second stage in response to which the probe is energized. The foot pedal may include at least one of a distance sensor or a contact sensor to operate in the first stage. The controller may be further configured to analyze the video to identify a critical structure of the tissue. The controller may be further configured to output the virtual representation of the probe based on a distance of the probe to the critical structure. The controller may be also configured to determine the distance of the probe to the critical structure based on at least one of the video or kinematics data of the first robotic arm and the second robotic arm. The controller may be additionally configured to highlight the critical structure. The monitor may be also configured to display the virtual representation in response to a user command.

According to a further embodiment of the present disclosure, a method for controlling deployment of a probe of a surgical instrument is disclosed. The method includes capturing a video of a tissue and a surgical instrument, the surgical instrument including an end effector assembly configured to treat the tissue. The surgical instrument also includes a probe adapted to connect to a source of energy. The probe is movable from a retracted position to a deployed position relative to the end effector assembly. The method also includes displaying on a monitor the video of the tissue and the surgical instrument, analyzing, at a controller, the video to identify a critical structure of the tissue and displaying on the monitor the virtual representation of the probe in the deployed position based on a distance of the probe to the critical structure.

Implementations of the above embodiment may include one or more of the following features. According to one aspect of the above embodiment, the method may further include receiving a first input from a switch to move the probe to the deployed position. The method may also include receiving a second input from a switch to energize the probe. The method may additionally include displaying, on the monitor, a prompt to the user to at least one of deploy or energize the probe based on identity of the critical structure.

### BRIEF DESCRIPTION OF DRAWINGS

Various aspects and features of this disclosure are described hereinbelow with reference to the drawings wherein like numerals designate identical or corresponding elements in each of the several views.
FIG. 1 is a schematic illustration of a surgical robotic system including a control tower, a console, and one or more surgical robotic arms according to aspects of this disclosure;
FIG. 2 is a perspective view of a surgical robotic arm of the surgical robotic system of FIG. 1 according to aspects of this disclosure;
FIG. 3 is a perspective view of a setup arm with the surgical robotic arm of the surgical robotic system of FIG. 1 according to aspects of this disclosure;
FIG. 4 is a schematic diagram of a computer architecture of the surgical robotic system of FIG. 1 according to aspects of this disclosure;
FIG. 5 is a front, perspective view of a proximal portion of a multifunction surgical instrument provided in accordance with this disclosure and configured for mounting on a robotic arm of a surgical robotic system such as the surgical robotic system of FIG. 1;
FIG. 6 is a rear, perspective view of the proximal portion of the multifunction surgical instrument of FIG. 5;
FIGS. 7A and 7B are perspective views of a distal portion of the multifunction surgical instrument of FIG. 5 with an end effector assembly thereof disposed in aligned and articulated positions, respectively;
FIG. 7C is a perspective views of the distal portion of the multifunction surgical instrument of FIG. 5 with the end effector assembly disposed in an aligned position and a probe disposed in a deployed position;
FIGS. 8A and 8B are respective top and bottom perspective views of a portion of one of the jaw members of the end effector assembly of FIGS. 7A-7C;
FIG. 9 is a longitudinal, cross-sectional view of a proximal portion of the multifunction surgical instrument of FIG. 5;
FIG. 10 is a perspective view of a portion of the actuation assembly of the multifunction surgical instrument of FIG. 5;
FIGS. 11A-11D are side views of various probes configured for use with the end effector assembly of FIGS. 7A-7C;
FIGS. 12-16 are longitudinal, cross-sectional views of the end effector assembly of FIGS. 7A-7C, wherein jaw members of the end effector assembly are disposed in an approximated position and wherein a probe of the end effector assembly is illustrated progressively advancing from a retracted position to an extended position to a deployed position;
FIG. 17 is a flow chart of a method for deploying the probe according to an embodiment of the present disclosure;
FIG. 18 is a schematic diagram of a system for determining phases of a surgical procedure according to an embodiment of the present disclosure; and
FIG. 19 is an augmented image of a surgical site and a graphical user interface (GUI) including a virtual projection of the probe according to an embodiment of the present disclosure.

### DETAILED DESCRIPTION

This disclosure provides multifunction surgical instruments. As described in detail below, the multifunction surgical instruments of this disclosure may be configured for use with a surgical robotic system, which may include, for example, a surgical console, a control tower, and one or more movable carts having a surgical robotic arm coupled to a setup arm. The surgical console receives user inputs through one or more interface devices, which are interpreted by the control tower as movement commands for moving the surgical robotic arm. The surgical robotic arm includes a controller, which is configured to process the movement commands and to generate a torque command for activating one or more actuators of the robotic arm, which, in turn, move the robotic arm in response to the movement commands. Although described hereinbelow in connection with surgical robotic systems, the aspects and features of this disclosure may also be adapted for use with handheld multifunction surgical instruments such as, for example, endoscopic instruments and/or open instruments.

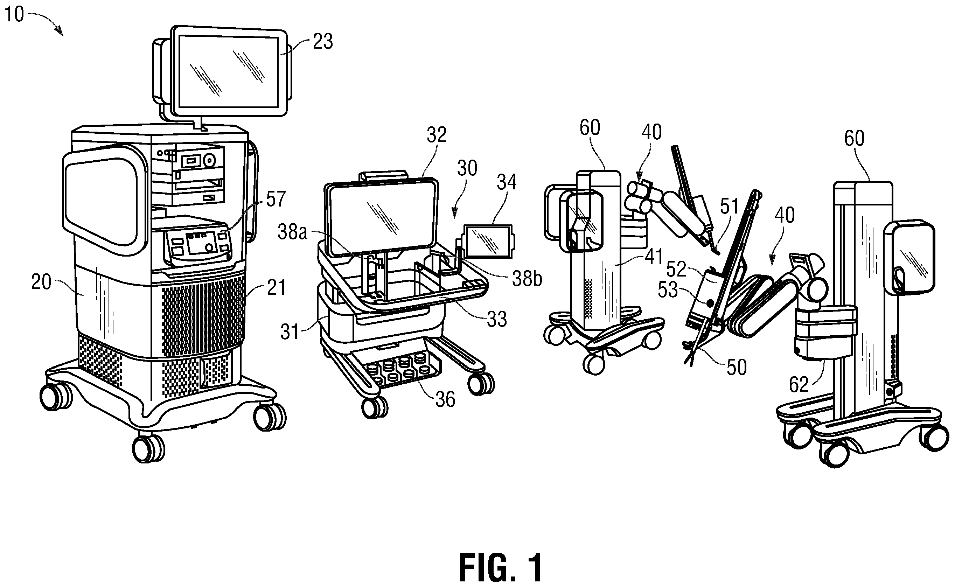

With reference to FIG. 1, a surgical robotic system 10 includes a control tower 20, which is connected to components of the surgical robotic system 10 including a surgical console 30 and one or more robotic arms 40. Each of the robotic arms 40 includes a surgical instrument 50 removably coupled thereto. Each of the robotic arms 40 is also coupled to a movable cart 60.

The one or more surgical instruments 50 may be configured for use during minimally invasive surgical procedures and/or open surgical procedures. In aspects, one of the surgical instruments 50 may be an endoscope, such as an endoscopic camera 51, configured to provide a video feed for the clinician. In further aspects, one of the surgical instruments 50 may be an energy based surgical instrument such as, for example, an electrosurgical forceps or ultrasonic sealing and dissection instrument configured to seal tissue by grasping tissue between opposing structures and applying electrosurgical energy or ultrasonic energy, respectively, thereto. In yet further aspects, one of the surgical instruments 50 may be a surgical stapler including a pair of jaws configured to clamp tissue, deploy a plurality of tissue fasteners, e.g., staples, through the clamped tissue, and/or to cut the stapled tissue. In still other aspects, one of the surgical instruments 50 may include an energizable element (e.g., a monopolar, bipolar, thermal, microwave, etc. element) configured to treat tissue. Suction and/or irrigation surgical instruments 50 are also contemplated. Other suitable surgical instruments 50 include the multifunction surgical instruments provided in accordance with this disclosure and described in detail hereinbelow.

Endoscopic camera 51, as noted above, may be configured to capture video of the surgical site. In such aspects, the surgical console 30 includes a first display 32, which displays a video feed of the surgical site provided by endoscopic camera 51, and a second display 34, which displays a user interface for controlling the surgical robotic system 10. The first and second displays 32 and 34 may be touchscreen graphical user interface (GUI) displays allowing for receipt of various user inputs.

The surgical console 30 also includes a plurality of user interface devices, such as foot pedals 36 and a pair of handle controllers 38a and 38b which are used by a clinician to remotely control robotic arms 40. The surgical console further includes an armrest 33 used to support clinician's arms while operating the handle controllers 38a and 38b.

The control tower 20 includes a display 23, which may be a touchscreen GUI, and provides outputs to the various GUIs. The control tower 20 also acts as an interface between the surgical console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgical console 30, in such a way that robotic arms 40 and the surgical instrument 50 execute a desired movement sequence in response to input from the foot pedals 36 and/or the handle controllers 38a and 38b.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by this disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth^{®} (an open wireless protocol for exchanging data over short distances, using short length radio waves, from fixed and mobile devices, creating personal area networks (PANs)), and/or ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor(s) operably connected to a memory, which may include one or more of volatile, non-volatile, magnetic, optical, quantum, and/or electrical media, such as read-only memory (ROM), random access memory (RAM), electrically-erasable programmable ROM (EEPROM), non-volatile RAM (NVRAM), or flash memory. The processor(s) may be any suitable processor(s) (e.g., control circuit(s)) adapted to perform operations, calculations, and/or set of instructions including, but not limited to, a hardware processor, a field programmable gate array (FPGA), a digital signal processor (DSP), a central processing unit (CPU), a microprocessor, a quantum processor, and combinations thereof. Those skilled in the art will appreciate that the processor may be substituted for by using any logic processor (e.g., control circuit) adapted to execute algorithms, calculations, and/or set of instructions.

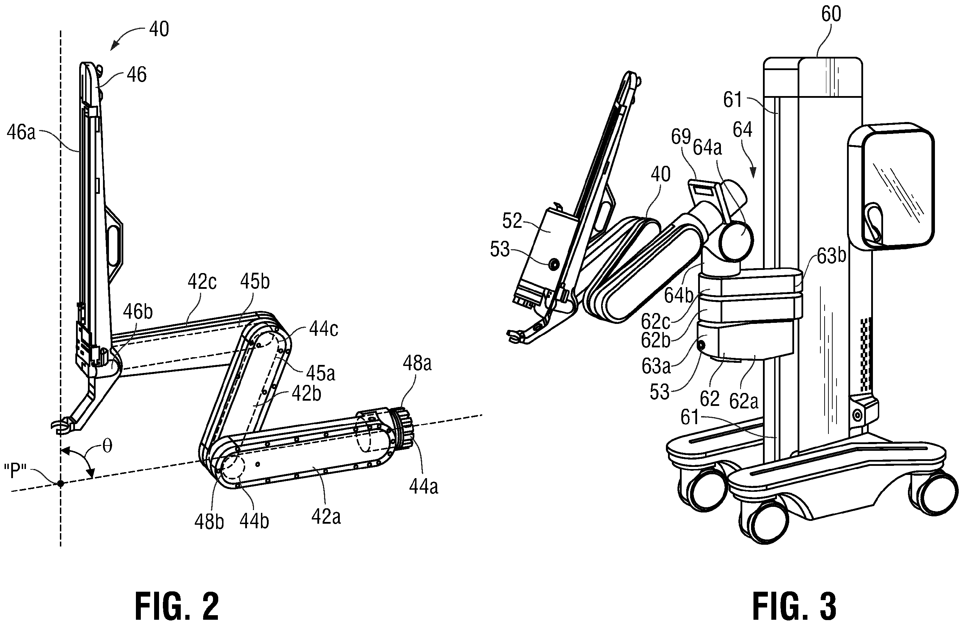

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40. The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62a and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In aspects, the robotic arm 40 may be coupled to the surgical table (not shown). The setup arm 62 may include controls (not shown) for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

With reference again to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and/or the camera 51 and is configured to move (e.g., rotate) and actuate the instrument 50 and/or the camera 51. IDU 52 transfers actuation forces from its actuators to the surgical instrument 50 to actuate components (e.g., end effectors) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c.

Referring momentarily to FIG. 3, the robotic arm 40 further includes a plurality of manual override buttons 53 disposed on the IDU 52 and the setup arm 62, which may be used in a manual mode. For example, the clinician may press one of the buttons 53 to move the component associated with that button 53.

Returning with reference to FIG. 2, the joints 44a and 44b include an actuator 48a and 48b configured to drive the joints 44a, 44b, 44c relative to each other through a series of belts 45a and 45b or other mechanical linkages such as drive rods, cables, levers, and/or the like. In particular, the actuator 48a is configured to rotate the robotic arm 40 about a longitudinal axis defined by the link 42a.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b such that the actuator 48b is configured to rotate each of the links 42b, 42c and the holder 46 relative to one another. More specifically, links 42b, 42c and the holder 46 are passively coupled to the actuator 48b which enforces rotation about a remote center point "P" that lies at an intersection of the first axis defined by the link 42a and the second axis defined by the holder 46. Thus, the actuator 48b controls the angle "θ" between the first and second axes allowing for orientation of the surgical instrument 50. Due to the interlinking of the links 42a, 42b, 42c and the holder 46 via the belts 45a and 45b, the angles between the links 42a, 42b, 42c and the holder 46 are also adjusted in order to achieve the desired angle "θ." In aspects, some or all of the joints 44a, 44b, 44c may include an actuator to obviate the need for mechanical linkages.

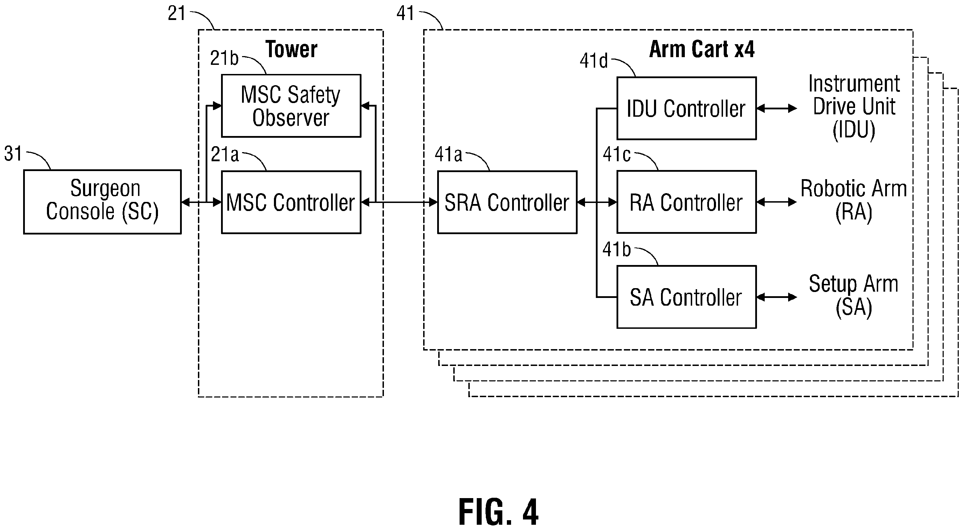

With reference to FIG. 4, in conjunction with FIG. 1, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and/or other inputs. The controller 21a processes these input positions to determine desired drive commands for each joint of the robotic arm 40 and/or the IDU 52 and communicates these to the computer 41 of the robotic arm 40. The controller 21a also receives the actual joint angles and uses this information to determine force feedback commands that are transmitted back to the computer 31 of the surgical console 30 to provide haptic or other feedback through the handle controllers 38a and 38b. The handle controllers 38a and 38b include one or more haptic feedback vibratory devices that output haptic feedback although visual, audible, and/or other feedback is also contemplated. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an IDU controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a communicates the actual joint angles back to the controller 21a.

The setup arm controller 41b controls each of joints 63a and 63b and the rotatable base 64 of the setup arm 62 and calculates desired motor movement commands (e.g., motor torque) for the pitch axis. The setup arm controller 41b also controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

With respect to control of the robotic arm 40, initially, a pose of the handle controller controlling the robotic arm 40, e.g., the handle controller 38a, is transformed into a desired pose of the robotic arm 40 through a hand eye transform function executed by the controller 21a. The hand eye function is embodied in software executable by the controller 21a or any other suitable controller of the surgical robotic system 10. The pose of the handle controller 38a may be embodied as a coordinate position and roll-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In aspects, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limiting mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

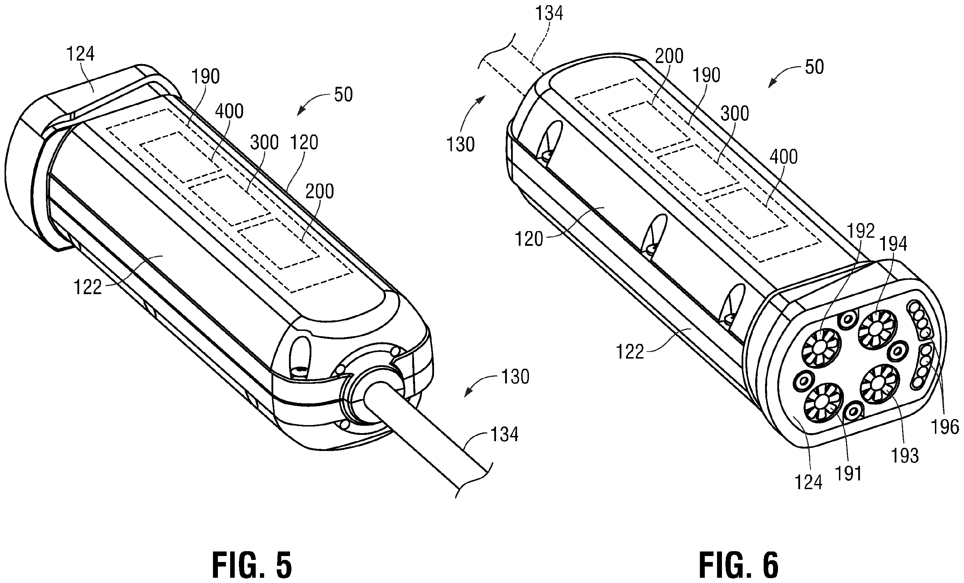

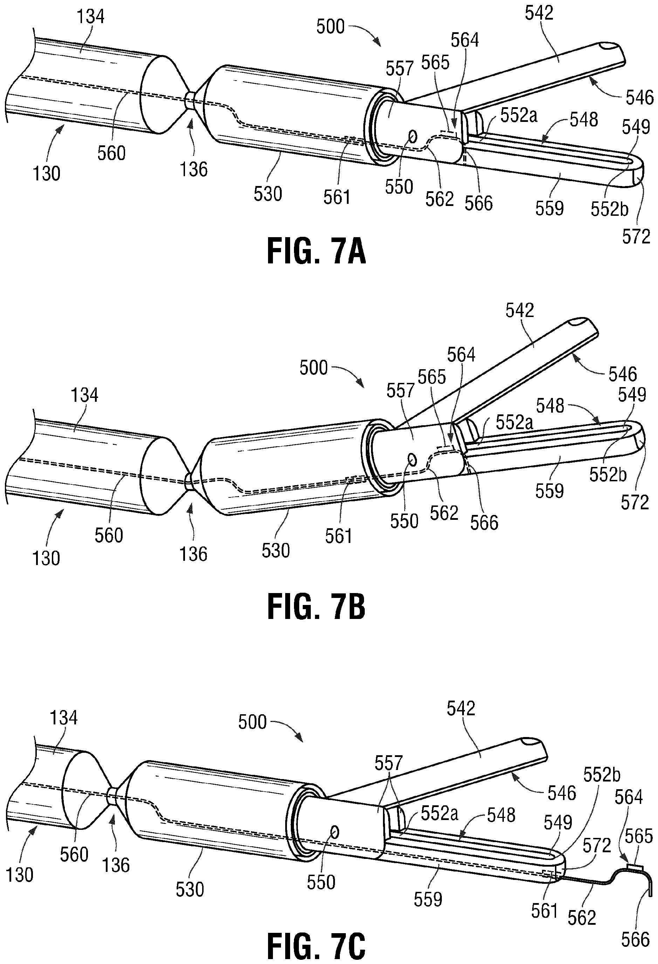

Turning to FIGS. 5-7C, a surgical instrument 50 provided in accordance with this disclosure generally includes a housing 120, a shaft assembly 130 extending distally from housing 120, an end effector assembly 500 extending distally from shaft assembly 130, and an actuation assembly 190 disposed within housing 120 and operably associated with end effector assembly 500. Instrument 50 is detailed herein as an articulating multifunction surgical instrument configured for use with a surgical robotic system, e.g., surgical robotic system 10 (FIG. 1). However, the aspects and features of instrument 50 provided in accordance with this disclosure, as detailed below, are equally applicable for use with other suitable surgical instruments and/or in other suitable surgical systems, e.g., motorized, other power-driven systems, and/or manually actuated surgical systems (including handheld instruments). Further, as an alternative to or in addition to articulation, instrument 50 may include a fixed shaft assembly 130, rotatable shaft assembly 130, malleable shaft assembly 130, combinations thereof, or any other suitable configuration to facilitate positioning end effector assembly 500 in a desired position and/or orientation relative to housing 120.

Housing 120 of instrument 50 includes a body 122 and a proximal face plate 124 that cooperate to enclose actuation assembly 190 therein. Proximal face plate 124 includes through holes defined therein through which four input actuators or couplers 191-194 of actuation assembly 190 extend. Proximal face plate 124 further mounts a plurality of electrical connectors 196 thereon to enable electrical connection of instrument 50 with a surgical robotic system, e.g., system 10 (FIG. 1), when instrument 50 is mounted on a robotic arm thereof, e.g., to enable communication of data, power, and/or control signals therebetween.

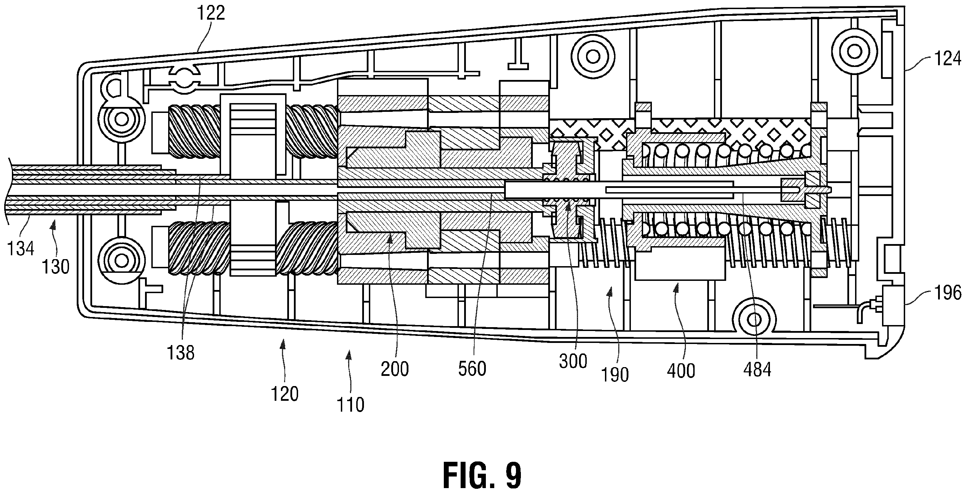

Shaft assembly 130 of instrument 50 includes a proximal shaft 134 and an articulating section 136 disposed between and interconnecting proximal section 134 with end effector assembly 500. Articulating section 136 includes one or more articulating components such as, for example, one or more links, pivots, joints, flexible bodies, etc. A plurality of articulation cables 138 (FIG. 9) or other suitable articulation actuators extend through articulating section 136. More specifically, articulation cables 138 (FIG. 9) may be operably coupled to end effector assembly 500 at the distal ends thereof and extend proximally through articulating section 136 of shaft assembly 130, proximal shaft 134 of shaft assembly 130, and into housing 120, wherein articulation cables 138 (FIG. 9) operably couple with an articulation sub-assembly 200 of actuation assembly 190 to enable selective articulation of end effector assembly 500 relative to proximal shaft 134 and housing 120, e.g., about at least one axis of articulation (yaw articulation, pitch articulation, or both yaw and pitch articulation, for example).

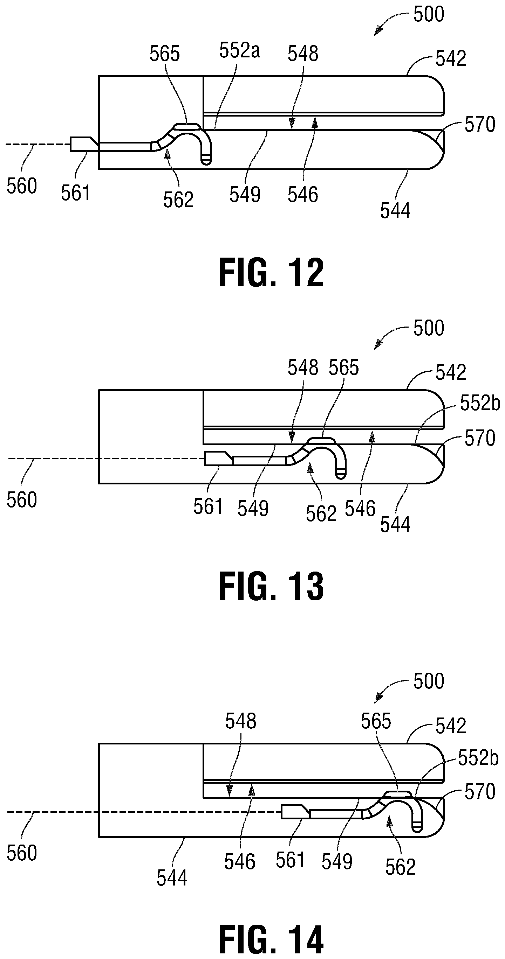

End effector assembly 500 includes a proximal body 530 operably engaged with articulating section 136 of shaft assembly 130. End effector assembly 500 further includes first and second jaw members 542, 544, respectively, pivotably coupled to one another about a pivot 550. Second jaw member 544 is fixed relative to proximal body 530 while first jaw member 542 is pivotable relative to second jaw member 544 and proximal body 530 between a spaced apart position (e.g., an open position of jaw members 542, 544) (FIGS. 7A-7C) and an approximated position (e.g., a closed position of jaw members 542, 544) (FIGS. 12-16) for grasping tissue between tissue contacting surfaces 546, 548 of jaw members 542, 544, respectively. As an alternative to this unilateral configuration, a bilateral configuration may be provided whereby both jaw members 542, 544 are pivotable relative to one another and proximal body 530.

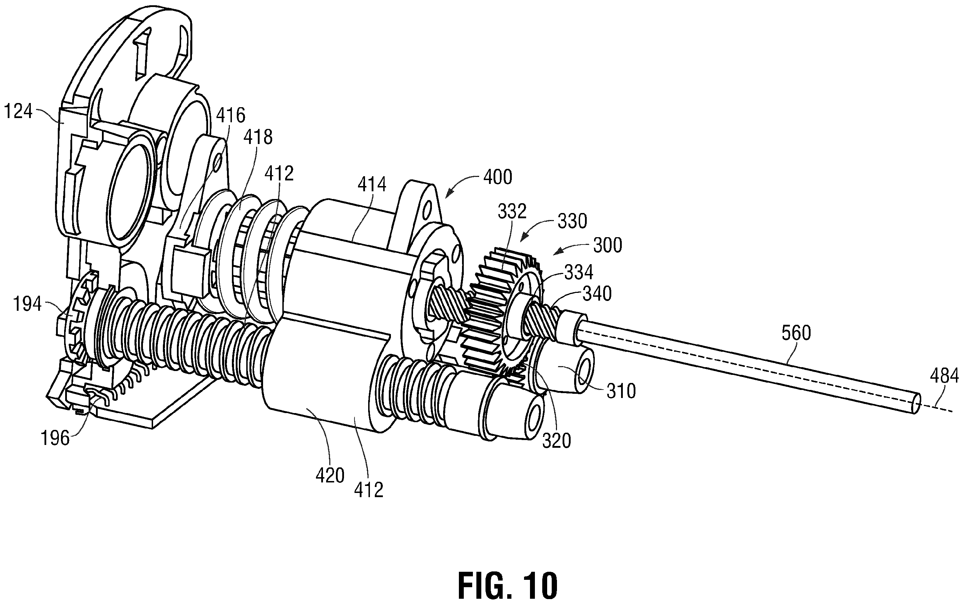

A jaw actuator 484 (FIGS. 9 and 10) is operably coupled to jaw members 542, 544 (e.g., via a cam-slot mechanism, one or more pulleys, closure-beam, etc.) such that longitudinal translation of jaw actuator 484 (FIGS. 9 and 10) relative to jaw members 542, 544 pivots jaw member 542 between the spaced-apart and approximated positions. Jaw actuator 484 extends proximally from end effector assembly 500 through shaft assembly 130 and into housing 120 wherein jaw actuator 484 (FIGS. 9 and 10) is operably coupled with a jaw drive sub-assembly 400 of actuation assembly 190 to enable selective actuation of jaw members 542, 544 between the spaced-apart and approximated positions to grasp tissue therebetween and apply a jaw force within an appropriate jaw force range, as detailed below.

Referring to FIGS. 7A-7C, tissue contacting surfaces 546, 548 of jaw members 542, 544, respectively, are at least partially formed from an electrically conductive material and are energizable to different potentials to enable the conduction of bipolar Radio Frequency (RF) electrical energy through tissue grasped therebetween, although tissue contacting surfaces 546, 548 may alternatively be configured to supply any suitable energy, e.g., thermal, microwave, light, ultrasonic, ultrasound, etc., through tissue grasped therebetween for energy based tissue treatment. Instrument 50 defines a pathway for conductors (not shown) through, along, and/or forming part of housing 120 and shaft 130 to end effector assembly 500 that may include lead wires, contacts, and/or electrically conductive components to enable electrical connection of tissue contacting surfaces 546, 548 of jaw members 542, 544, respectively, to an energy source, e.g., an electrosurgical generator 57 (FIG. 1), for supplying energy to tissue contacting surfaces 546, 548 to treat, e.g., seal, tissue grasped between tissue contacting surfaces 546, 548.

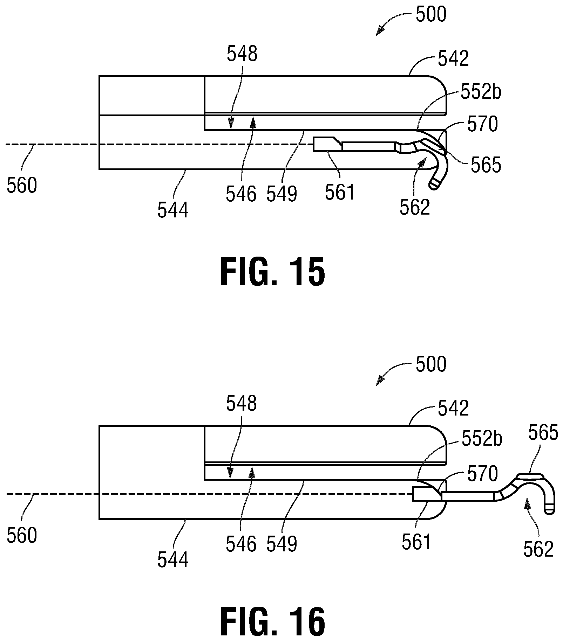

A longitudinally extending channel 549 is defined through tissue contacting surface 548 of jaw member 544. In aspects, a corresponding longitudinally extending channel (not shown) is defined through tissue contacting surface 546 of jaw member 542. The channel(s) 549 is configured to permit translation of a probe 562 therethrough. More specifically, a probe actuator 560 extending from housing 120 (see FIGS. 9 and 10) through shaft 130 to end effector assembly 500 is coupled to probe 562 to enable selective translation of probe 562 relative to jaw member 542 from a retracted position (FIGS. 7A-7C and 12), wherein probe 562 is disposed proximally of or at the proximal ends of tissue contacting surfaces 546, 548 of jaw members 542, 544, and an extended position (FIG. 14), wherein probe 562 extends through channel(s) 549 and between jaw member 542, 544, to cut (and/or otherwise treat) tissue grasped between tissue contacting surfaces 546, 548 of jaw members 542, 544, respectively. Probe actuator 560 is operably coupled to a probe drive sub-assembly 300 of actuation assembly 190 (see FIGS. 9 and 10) at a proximal end thereof and to probe 562 at a distal end thereof to enable the selective actuation (e.g., translation) of probe actuator 560 to, in turn, translate probe 562 between the retracted and extended positions. Probe 562 is further movable, via actuation of probe actuator 560, from the extended position (FIG. 14) to a deployed position (FIGS. 7C and 16), wherein at least a portion of probe 562 extends distally from jaw member 544, to enable probe 562 to cut (and/or otherwise treat) tissue positioned distally of jaw member 544. Probe actuator 560 may be joined to probe 562 in any suitable manner such as, for example, via welding or crimping, and, in aspects, a ferrule 561 may be provided at the interface between probe actuator 560 and probe 562 to facilitate the joining of probe actuator 560 and probe 562.

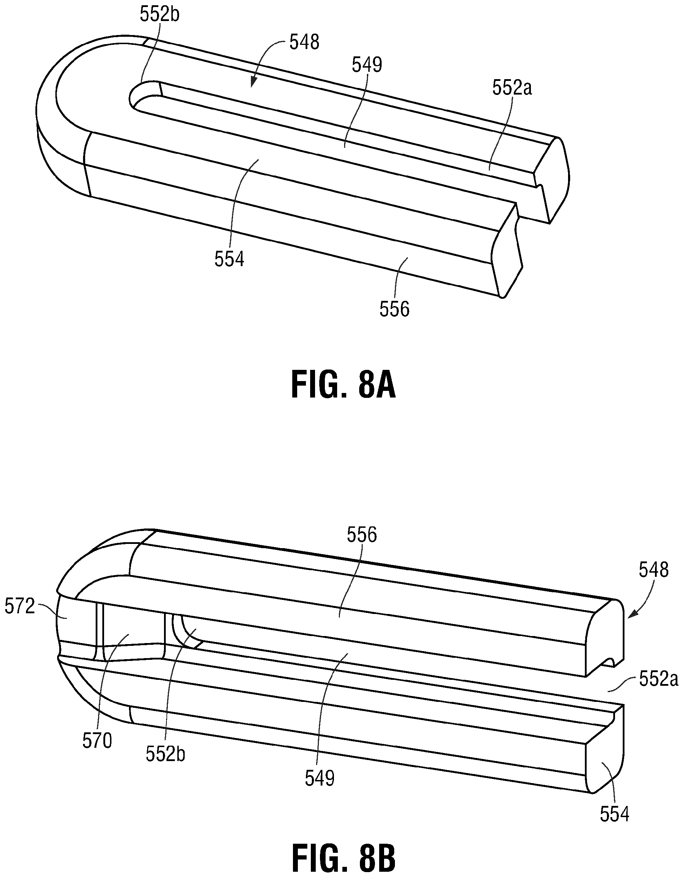

With reference to FIGS. 8A and 8B, in conjunction with FIGS. 7A-7C, longitudinally extending channel 549 of tissue contacting surface 548 of jaw member 544 includes an open proximal end 552a and a closed distal end 552b such that tissue contacting surface 548 defines a generally U-shaped configuration. In aspects, jaw member 544 includes a tissue contacting plate 554 defining tissue contacting surface 548 and an insert 556 supporting tissue contacting plate 554. In such aspects, insert 556 may be formed from an electrically insulative material while tissue contacting plate 554 is formed from an electrically conductive material. Insert 556 may be supported on a structural body 557 of jaw member 544 and, in aspects, insert 556, structural body 557, and a portion of tissue contacting plate 554 may be surrounded by a jaw housing 559, e.g., overmolded or otherwise disposed about these components to retain these components of jaw member 544 in position relative to one another.

In other aspects, tissue contacting plate 554 and insert 556 are monolithically formed as a single component, e.g., formed from an electrically conductive material. In such aspects, tissue contacting plate 554 and insert 556 may function as the structural body 557 of jaw member 544, or jaw member 544 may include a separate structural body 557 supporting tissue contacting plate 554 and insert 556 thereon. In either configuration, jaw housing 559 may also be provided, similarly as detailed above.

Continuing with reference to FIGS. 8A and 8B, in conjunction with FIGS. 7A-7C, tissue contacting plate 554 and/or insert 556 defines a ramp 570 on an underside thereof, e.g., opposite tissue contacting surface 548. Ramp 570 may include one or more angled surfaces, one or more curved surfaces, and/or any other configuration that facilitates guidance of probe 562 from the extended position (FIG. 14) to the deployed position (FIG. 16). More specifically, ramp 570 is configured to deflect probe 562 as probe 562 is advanced from the extended position (FIG. 14) towards the deployed position (FIG. 16) such that probe 562 ducks under closed distal end 552b of tissue contacting surface 548, extends through a passageway 572 (see also FIGS. 7A-7C) defined at the distal tip of jaw member 544, and deploys distally from passageway 572 and the distal tip of jaw member 544 to the deployed position (FIG. 16). Passageway 572 may be defined through insert 556, structural body 557, and/or jaw housing 559. Passageway 572 may define a tunnel extending from longitudinally extending channel 549 to an aperture at the distal tip of jaw member 544 and may define any suitable cross-sectional configuration, e.g., rectangular (or other polygonal shape), circular, oval, etc. In aspects where ferrule 561 is provided at the interface between probe actuator 560 and probe 562, ferrule 561 may be configured for abutment with or at least partial receipt within passageway 572 in complementary fit engagement in the deployed position (FIG. 16) of probe 562 such that additional structural support and resistance to splay of the deployed probe 562 is provided.

Referring to FIGS. 5-7C, 9, and 10, actuation assembly 190 is configured to operably interface with a surgical robotic system, e.g., system 10 (FIG. 1), when instrument 50 is mounted on a robotic arm thereof, to enable robotic operation of actuation assembly 190 to provide some or all of the above-detailed functionality. That is, surgical robotic system 10 (FIG. 1) selectively provides inputs, e.g., rotational inputs to input actuators or couplers 191-194 of actuation assembly 190 to: actuate articulation sub-assembly 200 to articulate end effector assembly 500 about at least one axis; actuate jaw drive sub-assembly 400 to manipulate jaw members 542, 544; actuate probe drive sub-assembly 300 to advance probe 562 between jaw members 542, 544; and/or further actuate probe drive sub-assembly 300 to deploy probe 562 from jaw member 544.

The above-noted five (5) functions are enabled by the only four (4) inputs to instrument 50: a first of the input actuators or couplers 191 enables articulation of end effector assembly 500 about a first axis of articulation (e.g., pitch articulation) to orient end effector assembly 500 in a first manner; a second of the input actuators or couplers 192 enables articulation of end effector assembly 500 about a second axis (e.g., perpendicular to the first axis) of articulation (e.g., yaw articulation) to orient end effector assembly 500 in a second manner; a third of the input actuators or couplers 193 enables actuation of probe drive sub-assembly 300 to both translate probe 562 between jaw members 542, 544 to treat tissue grasped between jaw members 542, 544 and deploy probe 562 from jaw member 544 to treat tissue disposed distally of jaw member 544; and a fourth of the input actuators or couplers 194 enables actuation of jaw drive sub-assembly 400 to open and close jaw members 542, 544 to release and grasp tissue.

Referring in particular to FIGS. 9 and 10, jaw drive sub-assembly 400, in aspects, includes a lead screw 410 operably coupled to fourth input actuator or coupler 194 and configured to rotate in response to a rotational input received at fourth input 194, a collar 412 threadingly engaged about lead screw 410 such that rotation of lead screw 410 translates collar 412 along lead screw 410, a first drive body 414 attached to (e.g., formed with, fixed on, or otherwise mechanically engaged with) collar 412 such that translation of collar 412 similarly translates first drive body 414, a second drive body 416 attached to (e.g., formed with, fixed on, or otherwise mechanically engaged with) jaw actuator 484 such that translation of second drive body 416 similarly translates jaw actuator 484, and a spring 418 (e.g., a compression coil spring) disposed between first and second drive bodies 414, 416.

As a result of the above-detailed configuration of jaw drive sub-assembly 400, a force-limiting feature is realized whereby the force applied to tissue grasped between jaw members 542, 544 is regulated. More specifically, during the initial movement of jaw member 542 towards jaw member 544 from the spaced-apart position towards the approximated position to grasp tissue between tissue contacting surfaces 546, 548, the rotational input received at fourth input 194 rotates lead screw 410 to translate collar 412, thereby translating first drive body 414 towards spring 418 to, in turn, urge spring 418 into second drive body 416 to move second drive body 416, thus translating jaw actuator 484 to pivot jaw member 542 towards jaw member 544. However, when the force applied to tissue grasped between jaw members 542, 544 exceeds a threshold, rather than spring 418 transferring motion to second drive body 416, spring 418 is compressed allowing second drive body 416 to remain stationary (and, thus, the force applied to grasped tissue does not exceed the threshold) despite further rotational input received at fourth input 194 to rotate lead screw 410, translate collar 412, and translate first drive body 414. That is, spring 418 compresses to absorb the translation of first drive body 414 rather than imparting motion to second drive body 416. Accordingly, prior to reaching the jaw force limit, first drive body 414, spring 418, second drive body 416, and jaw actuator 484 move substantially in concert with one another while, after reaching the jaw force limit, second drive body 416 and jaw actuator 484 remain substantially stationary despite further movement of first drive body 414 and the resultant compression of spring 418.

Continuing with reference to FIGS. 9 and 10, probe drive sub-assembly 300 includes a shaft 310 operably coupled to third input actuator or coupler 193 towards a first end of shaft 310 such that shaft 310 is configured to rotate in response to a rotational input to third input actuator or coupler 193. Probe drive sub-assembly 300 further includes a spur gear 320 fixed about shaft 310 towards a second end of shaft 310 such that rotation of shaft 310 rotates spur gear 320 in the same manner. Spur gear 320 is disposed in meshed engagement with an external spur gear 332 of a compound gear 330. Compound gear 330 further includes an internal lead nut 334 disposed about and in meshed engagement with a lead screw 340. Lead screw 340, in turn is engaged with probe actuator 560. As a result of the above-detailed configuration, a rotational input to third input actuator or coupler 193 rotates shaft 310 and spur gear 320 to thereby rotate compound gear 330 such that lead screw 340 is translated through and relative to compound gear 330 to thereby move probe actuator 560 proximally or distally. As noted above, probe actuator 560 is coupled to probe 562 (FIGS. 7A-7C). Thus, an appropriate input to third input actuator or coupler 193 may be utilized to translate probe 562 between jaw members 542, 544 (see FIGS. 12-14) and deploy probe 562 from jaw member 544 (see FIGS. 14-16). Probe actuator 560 may include any component or combination of components (e.g., shafts cables, linkages, etc.) to operably couple lead screw 340 and probe 562 with one another.

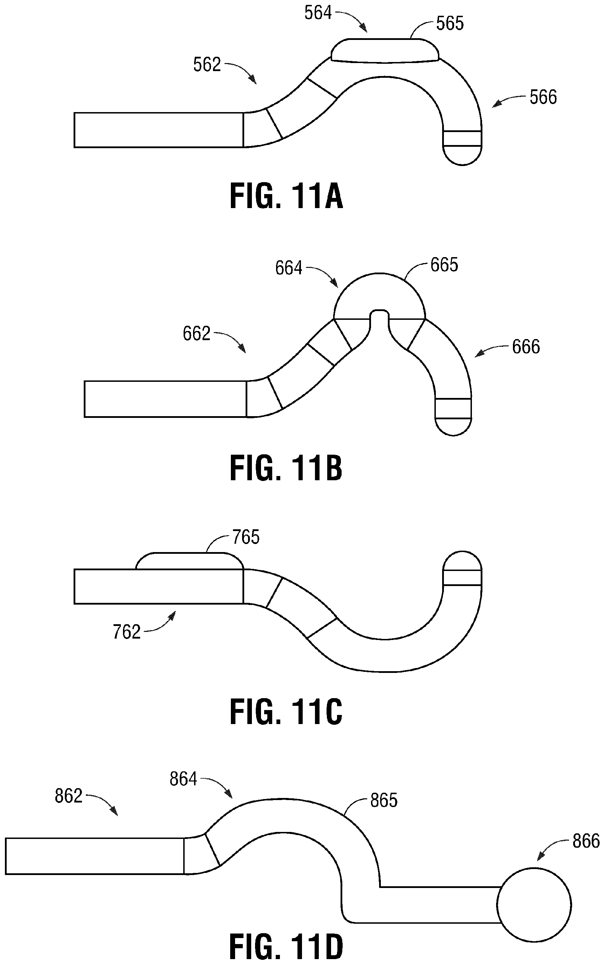

Turning to FIGS. 11A-11D, in conjunction with FIGS. 7A-7C, various probes 562, 662, 762, 862 (FIGS. 11A-11D, respectively) configured for use with surgical instrument 50 (FIG. 5) and, in particular, end effector assembly 500 (FIGS. 7A-7C) thereof are shown. Probes 562, 662, 762, 862 are configured to be energized with any suitable energy, e.g., RF (monopolar or bipolar), ultrasonic, thermal, light-energy, etc. For example, probes 562, 662, 762, 862 may connect to an electrosurgical generator 57 to enable the conduction of monopolar RF energy from probes 562, 662, 762, 862 to tissue to treat tissue while energy is returned to the electrosurgical generator 57 to complete the electrosurgical circuit via a remote return device (not shown), e.g., a return pad. Additionally or alternatively, probes 562, 662, 762, 862 may connect to the electrosurgical generator 57 to enable probes 562, 662, 762, 862 to be charged to a first electrical potential while the tissue contacting surface 546, 548 of either or both jaw members 542, 544, respectively, is charged to a second, different electrical potential to establish an electrical potential gradient for conducting RF energy between probes 562, 662, 762, 862 and either or both of jaw members 542, 544 and through tissue disposed therebetween to treat tissue with bipolar RF energy.

Referring initially to FIG. 11A, in conjunction with FIGS. 7A-7C, probe 562 is shown defining a question mark or hook configuration wherein the inner or concave portion of the hook is facing downwardly while the closed or convex portion of the hook is facing upwardly. Probe 562 includes a first portion 564 configured to treat tissue grasped between jaw members 542, 544 and a second portion 566 configured to treat tissue positioned distally of jaw members 542, 544. First portion 564 may, for example, be a feature of or disposed on the closed or convex portion of the hook. More specifically, as shown in FIG. 11A, probe 562 includes a fin 565 extending along a portion of and protruding upwardly from the closed or convex portion of the hook. Referring also to FIGS. 12-14, fin 565 is positioned such that, as probe 562 is translated through longitudinally extending channel 549 and along tissue contacting surface 548 of jaw member 544, fin 565 at least partially protrudes from tissue contacting surface 548 of jaw member 544 towards tissue contacting surface 546 of jaw member 542, thus enabling fin 565 to contact tissue grasped between jaw members 542, 544. Thus, with probe 562 energized and translating through longitudinally extending channel 549 from the retracted position (FIG. 12) towards the extended position (FIG. 14), fin 565 is urged through tissue to electromechanically cut tissue (via the mechanical movement of fin 565 relative to tissue and the energization of probe 562). In aspects, tissue grasped between jaw members 542, 544 is first sealed via the conduction of bipolar RF energy between tissue contacting surfaces 546, 548 and through the grasped tissue and is subsequently cut via the energization (in a monopolar or bipolar RF configuration) and translation of probe 562 from the retracted position (FIG. 12) towards the extended position (FIG. 14) and through the (previously sealed) tissue. In other aspects, previously unsealed tissue grasped between jaw members 542, 544 may be simultaneously or near simultaneously coagulated (or sealed) and cut via the above-noted energization and translation of probe 562. In yet other configurations, probe 562 may be utilized to only cut (or otherwise treat) tissue, e.g., in the absence of tissue sealing. Fin 565 may define a blunt configuration, e.g., rounded surfaces, to inhibit or reduce mechanical tissue cutting and to reduce current concentrations, although other configurations are also contemplated including angled or pointed surfaces to facilitate mechanical cutting and/or focus energy.

Continuing with reference to FIG. 11A, in conjunction with FIGS. 7A-7C, second portion 566 is configured to enable tissue treatment via energization of probe 562 (and, in aspects, movement of end effector assembly 500 relative to tissue) when probe 562 is disposed in the deployed position (see FIGS. 7C and 16). More specifically, with probe 562 energized, second portion 566 may be moved relative to tissue (e.g., via movement of end effector assembly 500 relative to tissue) to cut tissue, score tissue, spot coagulate tissue, separate tissue, perform an otomy, etc. In aspects, probe 562 is utilized in a monopolar RF configuration in the deployed position (see FIGS. 7C and 16) and in a bipolar RF configuration together with one or both of tissue contacting surfaces 546, 548 when moving between the retracted and extended positions (see FIGS. 12-14).

Turning to FIG. 11B, in conjunction with FIGS. 7A-7C, another probe 662 provided in accordance with this disclosure is shown. Probe 662 is similar to and may include any of the features of probe 562 (FIG. 11A) detailed above; thus, only differences between probe 662 and probe 562 (FIG. 11A) are described in detail below while similarities are omitted or only summarily described.

Probe 662 defines a question mark or hook configuration wherein the inner or concave portion of the hook is facing downwardly while the closed or convex portion of the hook is facing upwardly. Probe 662 includes a first portion 664 and a second portion 666. First portion 664 is configured as an upwardly-protruding hump 665 defined by an upwardly-protruding excursion from the closed or convex portion of the hook of probe 662. Hump 665 is positioned such that, as probe 662 is translated through longitudinally extending channel 549 and along tissue contacting surface 548 of jaw member 544, hump 665 at least partially protrudes from tissue contacting surface 548 of jaw member 544 towards tissue contacting surface 546 of jaw member 542, thus enabling hump 665 to contact and treat tissue grasped between jaw members 542, 544 (see FIGS. 7A and 12-14). Although hump 665 is shown defining a semi-circular configuration, other suitable configurations of hump 665 including one or more curvatures and/or angles are also contemplated.

FIG. 11C illustrates another probe 762 provided in accordance with this disclosure. Probe 762 is similar to and may include any of the features of probe 562 (FIG. 11A) except that probe 762 defines a question mark or hook configuration wherein the inner or concave portion of the hook is facing upwardly while the closed or convex portion of the hook is facing downwardly. Probe 762 also differs from probe 562 (FIG. 11A) in that fin 765 of probe 762 is positioned proximal to the hook portion of probe 762. As an alternative or in addition to providing fin 765, probe 762 may be configured such that a free distal end of the hook configuration is elongated to protrude from tissue contacting surface 548 of jaw member 544 towards tissue contacting surface 546 of jaw member 542, thus enabling the free distal end to be utilized to contact and treat tissue grasped between jaw members 542, 544 (see FIGS. 7A and 12-14).

FIG. 11D illustrates yet another probe 862 provided in accordance with this disclosure. Probe 862 is similar to and may include any of the features of probes 562, 662, 762 (FIGS. 11A-11C) except as explicitly contradicted below. Probe 862 includes a first portion 864 defined as a hump 865, although other configurations are also contemplated, and a second portion 866. Second portion 866 may define a ball end (as shown) or any other suitable configuration to facilitate tissue treatment such as, for example, a pointed end, a straight probe, an angled probe, a spatula, an S-curved element, a U-shaped element, etc. Alternatively or additionally, probe 862 (including both first and second portions 864, 866) may define any suitable configuration, for example, a hook, pointed end, a straight probe, an angled probe, a spatula, an S-curved element, a U-Shape, a D-shape, a loop, etc.

Turning to FIGS. 12-16, use of probe 562 of end effector assembly 500 and, more specifically, movement of probe 562 from the retracted position (FIG. 12) to the extended position (FIG. 14) and, subsequently, to the deployed position (FIG. 16) is detailed. Initially, as shown in FIG. 12, probe 562 is disposed in the retracted position wherein probe 562 does not protrude between tissue contacting surfaces 546, 548 or protrudes minimally (e.g., less than 10% of the length of tissue contacting surface 548) between tissue contacting surfaces 546, 548 of jaw members 542, 544 (in either the spaced-apart or approximated positions). In this position of probe 562, jaw members 542, 544 may be utilized to grasp and seal tissue, similarly as detailed above. In aspects, probe 562 may be moved from the retracted position only when jaw members 542, 544 are disposed in the approximated position (e.g., via mechanical and/or software stops); in other aspects, probe 562 may be moved from the retracted position regardless of the position of jaw members 542, 544.

With reference to FIGS. 12-14, in order to move probe 562 from the retracted position (FIG. 12) to the extended position (FIG. 14), probe drive sub-assembly 300 (FIGS. 5, 6, 9, and 10) is actuated, e.g., via a rotational input to third input actuator 193 (FIG. 6), to thereby advance probe actuator 560 distally. In conjunction with actuation of probe drive sub-assembly 300 (FIGS. 5, 6, 9, and 10), energy is supplied to probe 562 (and, in bipolar configurations, tissue contacting surface 546 and/or tissue contacting surface 548), to energize probe 562. As probe actuator 560 is advanced distally, the energized probe 562 is moved into open proximal end 552a of longitudinally extending channel 549 of tissue contacting surface 548 of jaw member 542 (if not already partially disposed within longitudinally extending channel 549) and distally through longitudinally extending channel 549. As noted above, during this movement of probe 562 through longitudinally extending channel 549, fin 565 protrudes above tissue contacting surface 548 such that fin 565 is moved into contact with and, through tissue grasped between tissue contacting surfaces 546, 548 of jaw members 542, 544 respectively, thereby cutting (and/or otherwise treating) the tissue. In the extended position (FIG. 14) of probe 562, in aspects, fin 565 extends to close approximation with closed distal end 552b of longitudinally extending channel 549 (e.g., within 10% of the length of longitudinally extending channel 549), thus enabling cutting of tissue along substantially the entire length of longitudinally extending channel 549.

With reference to FIGS. 14-16, in order to move probe 562 from the extended position (FIG. 14) to the deployed position (FIG. 16), probe drive sub-assembly 300 (FIGS. 5, 6, 9, and 10) is further actuated, e.g., via a rotational input to third input actuator 193 (FIG. 6), to thereby advance probe actuator 560 further distally to, in turn, urge probe 562 further distally. As probe 562 is urged further distally, rather than fin 565 contacting closed distal end 522b of longitudinally extending channel 549, probe 562 contacts ramp 570 within jaw member 544 such that ramp 570 deflects probe 562 and guides probe 562 to duck under closed distal end 552b of tissue contacting surface 548, extend through passageway 572 (see also FIGS. 7A-7C) defined at the distal tip of jaw member 544, and deploy distally from passageway 572 (see also FIGS. 7A-7C) and the distal tip of jaw member 544 to the deployed position (FIG. 16). In the deployed position, probe 562 may be energized (if not previously energized) for treating tissue similarly as detailed above. Further, in the deployed position, ferrule 561, if so provided, may be positioned in abutment or at least partially within passageway 572 to provide structural support to the deployed probe 562.

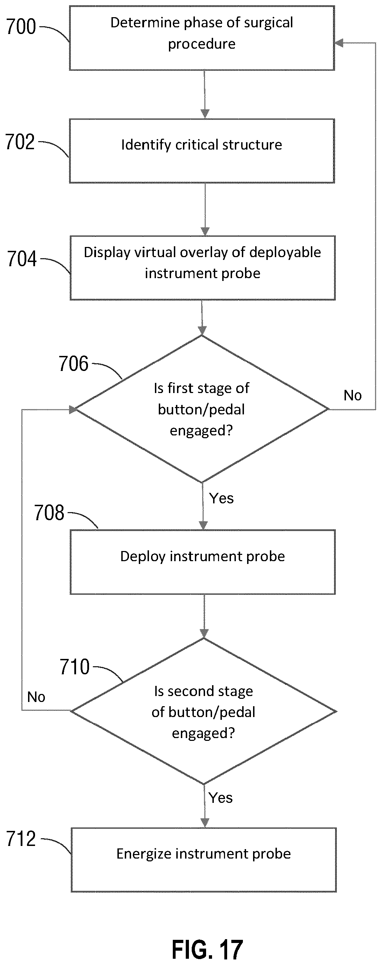

With reference to FIG. 17, a flow chart of a method for deploying the probe 562 may be implemented as software instructions stored in a non-transitory storage media (e.g., local storage, cloud storage, etc.) executable by one or more processors (e.g., controller 21a or any other suitable CPU, GPU, etc.). The method provides for automating some or all aspects of deployment of the probe 562 based on a specific phase of the surgical procedure. The surgical procedure may include multiple phases, and each phase may include one or more surgical actions. As used herein, the term "phase" represents a surgical event that is composed of a series of steps (e.g., closure). A "surgical action" may include an incision, a compression, a stapling, a clipping, a suturing, a cauterization, a sealing, dissection, or any other such actions performed to complete a phase in the surgical procedure. A "step" refers to the completion of a named surgical objective (e.g., hemostasis). During each step, certain surgical instruments 50 (e.g., forceps) are used to achieve a specific objective by performing one or more surgical actions.

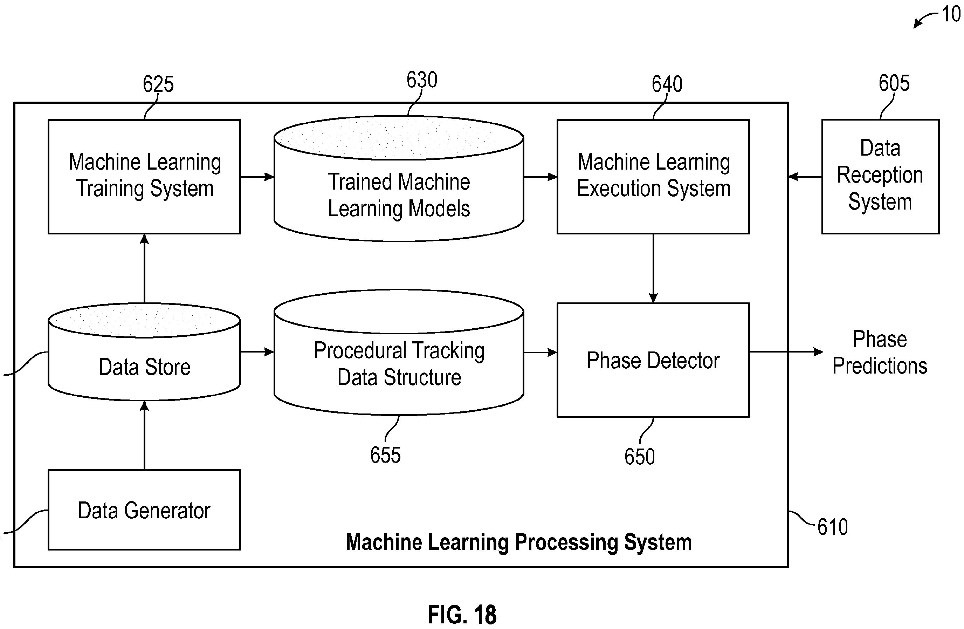

With reference to FIG. 18, the surgical robotic system 10 may include a machine learning (ML) processing system 610 that processes the surgical data using one or more ML models to identify one or more features, such as surgical phase, instrument, anatomical structure, etc., in the surgical data. The ML processing system 610 includes a ML training system 625, which may be a separate device (e.g., server) that stores its output as one or more trained ML models 660. The ML models 660 are accessible by a ML execution system 640. The ML execution system 640 may be separate from the ML training system 625, namely, devices that "train" the models are separate from devices that "infer," i.e., perform real-time processing of surgical data using the trained ML models 660.

System 10 includes a data reception system 605 that collects surgical data, including the video data and surgical instrumentation data. The data reception system 605 can include one or more devices (e.g., one or more user devices and/or servers) located within and/or associated with a surgical operating room and/or control center. The data reception system 605 can receive surgical data in real-time, i.e., as the surgical procedure is being performed.

The ML processing system 610, in some examples, may further include a data generator 615 to generate simulated surgical data, such as a set of virtual images, or record the video data from the video processing device 56, to train the ML models 660 as well as other sources of data, e.g., user input, arm movement, etc. Data generator 615 can access (read/write) a data store 620 to record data, including multiple images and/or multiple videos.

The ML processing system 610 also includes a phase detector 650 that uses the ML models to identify a phase within the surgical procedure ("procedure"). Phase detector 650 uses a particular procedural tracking data structure 655 from a list of procedural tracking data structures. Phase detector 650 selects the procedural tracking data structure 655 based on the type of surgical procedure that is being performed. In one or more examples, the type of surgical procedure is predetermined or input by user. The procedural tracking data structure 655 identifies a set of potential phases that may correspond to a part of the specific type of procedure.

In some examples, the procedural tracking data structure 655 may be a graph that includes a set of nodes and a set of edges, with each node corresponding to a potential phase. The edges may provide directional connections between nodes that indicate (via the direction) an expected order during which the phases will be encountered throughout an iteration of the procedure. The procedural tracking data structure 655 may include one or more branching nodes that feed to multiple next nodes and/or may include one or more points of divergence and/or convergence between the nodes. In some instances, a phase indicates a procedural action (e.g., surgical action) that is being performed or has been performed and/or indicates a combination of actions that have been performed. In some instances, a phase relates to a biological state of a patient undergoing a surgical procedure. For example, the biological state may indicate a complication (e.g., blood clots, clogged arteries/veins, etc.), pre-condition (e.g., lesions, polyps, etc.). In some examples, the ML models 660 are trained to detect an "abnormal condition," such as hemorrhaging, arrhythmias, blood vessel abnormality, etc.

The phase detector 650 outputs the phase prediction associated with a portion of the video data that is analyzed by the ML processing system 610. The phase prediction is associated with the portion of the video data by identifying a start time and an end time of the portion of the video that is analyzed by the ML execution system 640. The phase prediction that is output may include an identity of a surgical phase as detected by the phase detector 650 based on the output of the ML execution system 640. Further, the phase prediction, in one or more examples, may include identities of the structures (e.g., instrument, anatomy, etc.) that are identified by the ML execution system 640 in the portion of the video that is analyzed. The phase prediction may also include a confidence score of the prediction. Other examples may include various other types of information in the phase prediction that is output. The predicted phase may be used by the controller 21a to determine critical structures and whether to display various augmented or virtual reality representations to aid the surgeon. Critical structures include any vital tissue, e.g., artery, duct, etc. that dissection of which may cause harm to the patient

At step 700, the controller 21a via the ML processing system 610 determines the phase of the surgical procedure, in particular, the phase at which the probe 562 is about to be used. As stated above, the probe 562 may be used to cut tissue, score tissue, spot coagulate tissue, separate tissue, perform an otomy, etc. Laparoscopic cholecystectomy is an example of a surgical procedure during which the probe 562 may be used. This surgical procedure involves multiple dissection steps, e.g., incising peritoneum along the edge of the gallbladder on both sides to open up the hepatocystic triangle, dissection of cystic duct, cystic artery, etc. Laparoscopic cholecystectomy and other surgical procedures may have multiple phases during which the probe 562 may be used, thus, the method may be run continuously to determine dissection and resection phases.

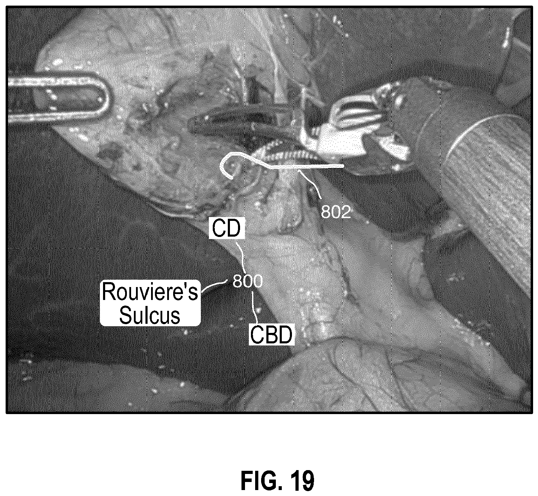

At step 702, the controller 21a via the ML processing system 610 identifies critical structure using computer vision and machine learning algorithms. In particular, the ML processing system 610 is configured to identify and, optionally, highlight critical structures. With reference to exemplary surgical procedure, i.e., cholecystectomy, critical structures that are identified include, the cystic duct, the cystic artery, the gallbladder, etc. The critical structures may be highlighted during the entire time the surgical site is visible by the camera 51, i.e., from very early stages of the procedure, before full exposure, up to full exposure, etc. This has the potential to assist and guide the surgeon, by keeping the focus on the hepatocystic triangle throughout the procedure, where the critical structures are found and where dissection is performed. As shown in FIG. 19, the cystic duct and cystic artery may be shown by using an indicator 800, which may be colored shading, confidence mapping, pins, borders outlining the structures, labels, etc.

At step 704, the controller 21a is configured to output a virtual representation 802 of the probe 562 to aid the surgeon in positioning the instrument 50 prior to using the probe 562 for dissection as shown in FIG. 19. The virtual representation 802 may appear to be the same as the probe 562 (e.g., same dimensions and shape) and may be transparent allowing for unobstructed visualization of the tissue and critical structures. The virtual representation 802 may be shown automatically based on detection of the phase and location of the instrument 50 relative to the critical structure. Distance (i.e., proximity) to the critical structure may be determined by the controller 21a using computer vision and machine learning algorithms of the video captured by the camera 51 and/or kinematic data of one or more of the robotic arm 40 holding the instrument 50 and the camera 51. In embodiments, the visual representation 802 may be shown in response to a manual user input, which may be a button on the handle controller 38a or 38b or one of the foot pedals 36. The virtual representation 802 may be generated by the controller 21a based on a 3D (e.g., CAD) model of the probe 562 and a reference point of the end effector assembly 500.

The foot pedals 36 or buttons may include two-stage mechanical switches, proximity or contact sensors to enable two-stage actuation. In embodiments where a two-stage mechanical switch is used, the first stage may be depression of a switch to a first distance and a second stage may be further depression to a second distance beyond the first distance. In embodiments, where a contact sensor is used, the first stage may be detecting contact of the switch without depression, which may be detected via capacitive sensors, and the second stage may be depression of the switch. In embodiments where proximity sensors are used, the first stage may be hovering of user's appendage or finger over the switch, which may be detected via proximity (e.g., optical) sensors, and the second stage may be depression of the switch. The first stage activation may be used to control deployment of the probe 562 whereas the second stage activation may be used to energize the probe 562 to enable cutting.

The engagement with the foot pedal 36 and/or the hand controller 38a or 38b is monitored by the controller 21a at step 706. If there is no engagement, the method returns to any of the previous steps 700-704. If there is first stage engagement, the controller 21a deploys the probe 562 at step 708 and then proceeds to monitor whether the activation switch is engaged in the second stage at step 710. If there is second stage engagement, the controller 21a energizes the probe 562 at step 712. If there is no second stage engagement, the controller 21a returns to step 706 to check if there is first stage engagement to determine whether to continue deploying the probe 562. The second stage verification of step 710 may have a timer such that if the second stage engagement does not occur within a predetermined period of time of first stage engagement, the controller 21a retracts the probe 562.

In embodiments, the foot pedal inputs may be replaced or complemented by GUI prompts asking the surgeon to display the virtual representation 802, deploy the probe 562, and then energize the probe 562. The prompts may be based on proximity to critical structures and the identity of the critical structure as described above with respect to automatic display of the virtual representation 802. Upon answering affirmatively to the prompts for displaying, deployment, and energization, etc. the corresponding functions are activated by the system 10.

It will be understood that various modifications may be made to the aspects and features disclosed herein. Therefore, the above description should not be construed as limiting, but merely as exemplifications of various configurations. Those skilled in the art will envision other modifications within the scope and spirit of the claims appended hereto.
The invention may be described by reference to the following numbered paragraphs:-
1. A surgical system comprising:
   a surgical instrument including:
      first and second jaw members, at least one of the first or second jaw members movable relative to the other of the first or second jaw members from a spaced apart position to an approximated position to grasp tissue therebetween, at least one of the first or second jaw members adapted to connect to a source of energy for conducting energy through tissue grasped between the first and second jaw members to treat the tissue; and
      a probe adapted to connect to a source of energy and movable from a retracted position to a deployed position;
   a camera configured to capture a video of a tissue and the surgical instrument;
   a controller configured to generate a virtual representation of the probe based on the video of the surgical instrument; and
   a monitor configured to display the video of the tissue and the surgical instrument, and the virtual representation of the probe in the deployed position.
2. The surgical system according to paragraph 1, further comprising a switch operable in a first stage in response to which the probe is moved to the deployed position.
3. The surgical system according to paragraph 2, wherein the switch is operable in a second stage in response to which the probe is energized.
4. The surgical system according to paragraph 1, wherein the controller is further configured to analyze the video to identify a critical structure of the tissue.
5. The surgical system according to paragraph 4, wherein the controller is further configured to output the virtual representation of the probe based on a distance of the probe to the critical structure.
6. The surgical system according to paragraph 4, wherein the controller is further configured to highlight the critical structure.
7. The surgical system according to paragraph 1, wherein the monitor is configured to display the virtual representation in response to a user command.
8. A surgical robotic system comprising:
   a first robotic arm controlling a surgical instrument including:
      first and second jaw members, at least one of the first or second jaw members movable relative to the other of the first or second jaw members from a spaced apart position to an approximated position to grasp tissue therebetween, at least one of the first or second jaw members adapted to connect to a source of energy for conducting energy through tissue grasped therebetween the first and second jaw members to treat the tissue; and
      a probe adapted to connect to a source of energy, the probe movable from a retracted position to a deployed position;
   a second robotic arm controlling a camera configured to capture a video of a tissue and the surgical instrument;
   a controller configured to generate a virtual representation of the probe based on the video of the surgical instrument; and
   a monitor configured to display the video of the tissue and the surgical instrument, and the virtual representation of the probe in the deployed position.
9. The surgical robotic system according to paragraph 8, further comprising a surgical console including a foot pedal operable in a first stage in response to which the probe is moved to the deployed position.
10. The surgical robotic system according to paragraph 9, wherein the foot pedal is operable in a second stage in response to which the probe is energized.
11. The surgical robotic system according to paragraph 9, wherein the foot pedal includes at least one of a distance sensor or a contact sensor to operate in the first stage.
12. The surgical robotic system according to paragraph 8, wherein the controller is further configured to analyze the video to identify a critical structure of the tissue.
13. The surgical robotic system according to paragraph 12, wherein the controller is further configured to output the virtual representation of the probe based on a distance of the probe to the critical structure.
14. The surgical robotic system according to paragraph 13, wherein the controller is further configured to determine the distance of the probe to the critical structure based on at least one of the video or kinematics data of the first robotic arm and the second robotic arm.
15. The surgical robotic system according to paragraph 12, wherein the controller is further configured to highlight the critical structure.
16. The surgical robotic system according to paragraph 8, wherein the monitor is configured to display the virtual representation in response to a user command.
17. A method for controlling deployment of a probe of a surgical instrument, the method comprising:
   capturing a video of a tissue and a surgical instrument including an end effector assembly and a probe adapted to connect to a source of energy, the probe movable from a retracted position to a deployed position relative to the end effector assembly;
   generating, at a controller, a virtual representation of the probe based on the video of the surgical instrument;
   displaying on a monitor the video of the tissue and the surgical instrument;
   analyzing, at a controller, the video to identify a critical structure of the tissue; and
   displaying on the monitor the virtual representation of the probe in the deployed position based on a distance of the probe to the critical structure.
18. The method according to paragraph 17, further comprising receiving a first input from a switch to move the probe to the deployed position.
19. The method according to paragraph 17, further comprising receiving a second input from a switch to energize the probe.
20. The method according to paragraph 17, further comprising:
   displaying, on the monitor, a prompt to the user to at least one of deploy or energize the probe based on identity of the critical structure.

## Claims

1. A surgical system comprising:
a surgical instrument including:
first and second jaw members, at least one of the first or second jaw members movable relative to the other of the first or second jaw members from a spaced apart position to an approximated position to grasp tissue therebetween, at least one of the first or second jaw members adapted to connect to a source of energy for conducting energy through tissue grasped between the first and second jaw members to treat the tissue; and
a probe adapted to connect to a source of energy and movable from a retracted position to a deployed position;
a camera configured to capture a video of a tissue and the surgical instrument;
a controller configured to generate a virtual representation of the probe based on the video of the surgical instrument; and
a monitor configured to display the video of the tissue and the surgical instrument, and the virtual representation of the probe in the deployed position.

2. The surgical system according to claim 1, further comprising a switch operable in a first stage in response to which the probe is moved to the deployed position.

3. The surgical system according to claim 2, wherein the switch is operable in a second stage in response to which the probe is energized.

4. The surgical system according to any preceding claim, wherein the controller is further configured to analyze the video to identify a critical structure of the tissue.

5. The surgical system according to claim 4, wherein the controller is further configured to output the virtual representation of the probe based on a distance of the probe to the critical structure.

6. The surgical system according to claim 4 or claim 5, wherein the controller is further configured to highlight the critical structure.

7. The surgical system according to any preceding claim, wherein the monitor is configured to display the virtual representation in response to a user command.

8. A surgical robotic system comprising:
a first robotic arm controlling a surgical instrument including:
first and second jaw members, at least one of the first or second jaw members movable relative to the other of the first or second jaw members from a spaced apart position to an approximated position to grasp tissue therebetween, at least one of the first or second jaw members adapted to connect to a source of energy for conducting energy through tissue grasped therebetween the first and second jaw members to treat the tissue; and
a probe adapted to connect to a source of energy, the probe movable from a retracted position to a deployed position;
a second robotic arm controlling a camera configured to capture a video of a tissue and the surgical instrument;
a controller configured to generate a virtual representation of the probe based on the video of the surgical instrument; and
a monitor configured to display the video of the tissue and the surgical instrument, and the virtual representation of the probe in the deployed position.

9. The surgical robotic system according to claim 8, further comprising a surgical console including a foot pedal operable in a first stage in response to which the probe is moved to the deployed position; preferably wherein the foot pedal is operable in a second stage in response to which the probe is energized.

10. The surgical robotic system according to claim 9, wherein the foot pedal includes at least one of a distance sensor or a contact sensor to operate in the first stage.

11. The surgical robotic system according to any of claims 8 to 10, wherein the controller is further configured to analyze the video to identify a critical structure of the tissue; preferably wherein the controller is further configured to output the virtual representation of the probe based on a distance of the probe to the critical structure.

12. The surgical robotic system according to claim 11, wherein the controller is further configured to determine the distance of the probe to the critical structure based on at least one of the video or kinematics data of the first robotic arm and the second robotic arm; preferably wherein the controller is further configured to highlight the critical structure.

13. The surgical robotic system according to any of claims 8 to 12, wherein the monitor is configured to display the virtual representation in response to a user command.

14. A method for controlling deployment of a probe of a surgical instrument, the method comprising:
capturing a video of a tissue and a surgical instrument including an end effector assembly and a probe adapted to connect to a source of energy, the probe movable from a retracted position to a deployed position relative to the end effector assembly;
generating, at a controller, a virtual representation of the probe based on the video of the surgical instrument;
displaying on a monitor the video of the tissue and the surgical instrument;
analyzing, at a controller, the video to identify a critical structure of the tissue; and
displaying on the monitor the virtual representation of the probe in the deployed position based on a distance of the probe to the critical structure.

15. The method according to claim 14, further comprising receiving a first input from a switch to move the probe to the deployed position; preferably further comprising receiving a second input from a switch to energize the probe; preferably further still comprising:
displaying, on the monitor, a prompt to the user to at least one of deploy or energize the probe based on identity of the critical structure.
